# EUROPEAN PATENT SPECIFICATION

(11) **EP 4 093 849 B1**
(45) Date of publication and mention of the grant of the patent: **29.05.2024**
(21) Application number: 21711633.4
(22) Date of filing: 22.01.2021
(51) Int. Cl.: C12N 1/12, C12R 1/89

(54) **STRAIN OF UNICELLULAR RED ALGAE, METHOD FOR OBTAINING UNICELLULAR RED ALGAE AND USE OF THE STRAIN FOR BIOREMEDIATION AND FOR THE PREPARATION OF BIOFUELS**
STAMM VON EINZELLIGEN ROTALGEN, VERFAHREN ZUR GEWINNUNG EINZELLIGER ROTALGEN UND VERWENDUNG DES STAMMES ZUR BIOSANIERUNG UND ZUR HERSTELLUNG VON BIOKRAFTSTOFFEN
SOUCHE D'ALGUES ROUGES UNICELLULAIRES, PROCÉDÉ D'OBTENTION D'ALGUES ROUGES UNICELLULAIRES ET UTILISATION DE LA SOUCHE POUR LA BIOREMÉDIATION ET POUR LA PRÉPARATION DE BIOCARBURANTS

(30) Priority: 24.01.2020 PL 43268420
(43) Date of publication of application: 30.11.2022
(73) Proprietor: Uniwersytet Warszawski, 00-927 Warszawa (PL)
(72) Inventor: BOROWSKA, Ewa, 42-440 Ogrodzieniec (PL); ABRAM, Mateusz, 88-100 Inowroclaw (PL); KARGUL, Joanna, 02-763 Warszawa (PL)
(74) Representative: Patpol Kancelaria Patentowa Sp. z o.o.
(86) International application number: PCT/IB2021/050512
(87) International publication number: WO 2021/149011

(56) References cited:
- WO-A1-2019/107385
- WO-A1-2020/071444
- JP-A- 2017 123 816
- MINODA A ET AL: "Improvement of culture conditions and evidence for nuclear transformation by homologous recombination in a red alga, Cyanidioschyzon merolae 10D", PLANT AND CELL PHSIOLOGY, OXFORD UNIVERSITY PRESS, UK, vol. 45, no. 6, 1 June 2004 (2004-06-01), pages 667-671, XP002693205, ISSN: 0032-0781, DOI: 10.1093/PCP/PCH087
- Motomichi Matsuzaki ET AL: "Genome sequence of the ultrasmall unicellular red alga Cyanidioschyzon merolae 10D", Nature, vol. 428, no. 6983, 8 April 2004 (2004-04-08), pages 653-657, XP055187910, DOI: 10.1038/nature02398
- HIROOKA SHUNSUKE ET AL: "Efficient open cultivation of cyanidialean red algae in acidified seawater", SCIENTIFIC REPORTS, vol. 10, no. 1, 1 December 2020 (2020-12-01), XP055802603, DOI: 10.1038/s41598-020-70398-z Retrieved from the Internet: URL:https://www.nature.com/articles/s41598 -020-70398-z.pdf>

## Description

### Technical field

The object of the invention is a novel strain of a unicellular red alga *Cyanidioschyzon merolae.*

The invention also relates to a use of the strain according to the invention for bioremediation and for preparation of biofuels.

### Background art

Acidic environment, i.e. with low pH, may occur naturally or be the result of human activity. It is not uncommon that acidic conditions produced by man are accompanied by severe environmental pollution (for example acidic mine outflows are considered one of the greatest environmental hazards). One of the possibilities of reducing environmental damage is using microorganisms in bioremediation processes and processing of harmful waste. However, such microorganisms must be able to grow in conditions with low pH.

Microorganisms for which optimal pH for growth is below 3 are referred to as acidophilic or acidophiles. Most of the known acidophile species belong to bacteria or archea. Low pH of the external environment enforces maintaining high pH gradient across the cell membrane. Various aspects of adapting microbial cells to such conditions have been described, such as transmembrane channel systems and pumps responsible for active transport of potassium cations, cell membranes with low permeability, increased synthesis of organic acids, variants of enzymes, which are stable at low pH, as well as presence of higher amounts of chaperones and repair proteins allowing to reduce damage to proteins and nucleic acids (see review in Austin and Dopson, Life in acid: pH homeostasis in acidophiles, Trends in Microbiology, 2007, vol. 15, issue 4). There is still however no complete understanding of the mechanisms responsible for cell survival and growth at high acidity, which is a serious obstacle while designing, modifying or creating new, biotechnologically useful strains with genetic engineering methods (Gumulya et al., In a Quest for Engineering Acidophiles for Biomining Applications: Challenges and Opportunities, Genes, 2018, 9, 116). Among others, strategies for modification of lactic acid bacteria or *E. coli* towards overexpression of chaperones, introducing exogenous metabolic pathways (e.g. trehalose biosynthesis) or maintaining cell membrane functionality under conditions of low pH are described (Wu, C.; Huang, J.; Zhou, R. Progress in engineering acid stress resistance of lactic acid bacteria. Appl. Microbiol. Biotechnol. 2014, 98, 1055-1063; Carvalho, A.L.; Cardoso, F.S.; Bohn, A.; Neves, A.R.; Santos, H. Engineering trehalose synthesis in Lactococcus lactis for improved stress tolerance. Appl. Environ. Microbiol. 2011, 77, 4189-4199.). Adaptive culture strategies are also known, involving a series of culture passages in order to obtain strains adapted to desired conditions (e.g., Ai, C.B.; McCarthy, S.; Eckrich, V.; Rudrappa, D.; Qiu, G.Z.; Blum, P. Increased acid resistance of the archaeon, Metallosphaera sedula by adaptive laboratory evolution. J. Ind. Microbiol. Biot. 2016, 43, 1455-1465), however the process is time-consuming and may take several years (McCarthy, S.; Johnson, T.; Pavlik, B.J.; Payne, S.; Schackwitz, W.; Martin, J.; Lipzen, A.; Keffeler, E.; Blum, P. Expanding the limits of thermoacidophily in the archaeon Sulfolobus solfataricus by adaptive evolution. Appl. Environ. Microbiol. 2016, 82, 857-867). In order to accelerate it, mutagenesis induced by e.g. UV radiation has been used.

For the purification of water from contaminants, such as heavy metals, the use of microalgae has been proposed in the art. Many species are adapted to capture metal ions from the environment, and their use has been postulated in water purification as a supplementary step to purification with physicochemical methods (Kaplan, Absorption and adsorption of heavy metals by microalgae, 2013, Handbook of Microalgal Culture: Applied Phycology and Biotechnology, 2nd edition, Richmond and Qiang Hu ed., John Wiley & Sons, Ltd). Since for the purposes of bioremediation it is necessary for the strain to maintain the ability to grow under difficult conditions, associated with presence of heavy metals in high concentrations, low pH, etc., it has been postulated to use microalgae isolated from environments with such conditions, e.g. with low pH, such as extremophilic green algae *Coccomyxa melkonianii* strains, which are capable of growing in a wide pH range from 4.0 to 8.0 (Soru et al. Behavior of the extremophile green alga Coccomyxa melkonianii SCCA 048 in terms of lipids production and morphology at different pH values, 2019, Extremophiles, 23:79-89). Also the possibility of using extremophilic microalgae in the biosynthesis of products useful in production of biofuels, food, ingredients for plastics, etc. was indicated (Soru et al., A novel investigation of growth and lipid production of the extremophile microalga Coccomyxa melkonianii SCCA 048 under the effect of different cultivation conditions: Experiments and modelling, 2018, Chemical Engineering Journal, https://doi.org/10.1016/j.cej.2018.12.049).

The unicellular red microalga *Cyanidioschyzon merolae* is able to grow under extreme conditions (low pH values and temperatures of 40 - 56°C), although the species has been shown to survive at temperatures of 35°C in water and 10°C in soil. Successful adaptation of this organism to growth at room temperature (25°C), lasting about 250 hours, has been described; however, it involved significant restructuring of the photosynthetic apparatus and a decrease in the dry mass of cells in the culture (Nikolova et al., Temperature-Induced Remodeling of the Photosynthetic Machinery Tunes Photosynthesis in the Thermophilic Alga Cyanidioschyzon merolae, 2017, Plant Physiology, vol. 174, pp. 35-46).

The present inventors obtained a novel strain of a unicellular red alga *Cyanidioschyzon merolae,* based on the strain NIES-1332, acquired from the Microbial Culture Collection from the National Institute for Environmental Studies (Tsukuba, Japan), wherein the novel strain of the invention is adapted to variable pH and capable of effective growth under pH conditions in the range of 2.5 to 5.5 without a buffering agent, and, when a buffering agent is used, in pH range of 5.5 - 6.7. It may be cultivated in a temperature range of 25 - 65°C, although the optimal range is 40 - 42°C. The newly obtained strain is capable of the biofilm formation on cotton fibers, in contrast to the control strain grown at pH 2.5. It has the potential for bioremediation of heavy metals and biofuels production, both in the form of a cell suspension and a biofilm, due to high survivability of this species in a natural environment rich in heavy metals and metalloids. Adapting the C. *merolae* strain for cultivation (both in the form of cell suspension and biofilm) at neutral pH significantly increases the application possibilities for this strain in bioremediation of heavy metals due to the increased sorption of selected heavy metals at elevated pH (Lee and Saunders 2003, *supra*)*.*

Thus, the object of the invention is a strain of the unicellular red alga *Cyanidioschyzon merolae,* deposited on the 25^{th} October 2019, in accordance with the requirements of The Budapest treaty in Banco Español de Algas, Marine Biotechnology Center, University of Las Palmas, Gran Canaria, Muelle de Taliarte s/n, 35214 Telde, Spain, under the number BEA_IDA_0073B. Viability of the deposited strain was confirmed on 17^{th} December 2019.

Also disclosed but not part of the invention is a method for obtaining unicellular red algae, including the steps of:
a) preculture of an acidophilic strain of unicellular red algae under conditions of low pH optimal for the growth of the strain;
b) first passage to a medium without a buffering agent with increased pH, with pH of such a diluted culture increasing by a minimum of 1 unit, wherein resulting OD₇₅₀ of such a diluted culture is ≥0.3;.
c) a series of passages to a medium without a buffering agent with increased pH, wherein in each consecutive step of diluting the dense culture with a fresh medium, the increase in pH of such diluted culture is maintained between 0.8 and 1.5 pH units;
d) at least one pH stabilizing passage with the addition of a buffering agent, wherein pH of the culture increases by between 0.3 and 0.8 pH units;
e) at least one pH stabilizing passage with the addition of a buffering agent at a higher concentration than in step d), wherein increase in the culture's pH of less than 0.5 pH unit is provided.

As a result of the method above, a final culture of unicellular red algae is obtained, with stable pH, higher than pH in step a), which was optimal for the acidophilic starting strain.

Also disclosed is the method above wherein in step b) the volume ratio of the diluted culture to the fresh medium without a buffering agent with increased pH is 1 : 4. For successive steps of adapting the cells to a higher pH, this ratio depends on the optical density of the donor culture and affects the period between successive passages. It is also possible to perform shorter growth stages of the culture between passages, while providing lower pH changes between passages. Despite the increased number of steps, such a process will provide a similar time for adapting the culture to the final target pH.

Also disclosed is the method above wherein the pH of the final culture after step e) is higher than acidic.

Also disclosed is the method above wherein the pH of the final culture after step e) is in the neutral range.

As understood herein, pH in the neutral range is pH in the range above 6.4 but below 7.2.

Also disclosed is the method above wherein pH of the final culture after step e) is stable and is 6.7 ± 0.3.

Also disclosed is the method above wherein the buffering agent is 2-morpholinoethanesulfonic acid.

Also disclosed is the method above wherein in step d) the buffering agent used is 2-morpholinoethanesulfonic acid at a concentration of 10 mM.

Also disclosed is the method above wherein in step e) the buffering agent used is 2-morpholinoethanesulfonic acid at a concentration of 20 mM.

Also disclosed but not part of the invention is a strain of unicellular red algae obtained with the method above.

Preferably, the strain is capable of effective growth under pH conditions in the range of 2.5 to 5.5 without a buffering agent, and in the range of pH 5.5 - 6.7 when a buffering agent is used. In the present application, effective growth is intended to mean an increase in biomass and metabolic activity, particularly including the level of lipid production, which is not statistically significantly reduced as compared to the starting strain used in step a).

The object of the invention is also use of the strain according to the invention for bioremediation.

In a preferred embodiment of the use of the strain for bioremediation according to the invention, the bioremediation comprises removal of heavy metals.

In a preferred embodiment of the use of the strain for bioremediation according to the invention, wastewater or groundwater is subjected to bioremediation.

The object of the invention is also use of the strain according to the invention to produce biofuels.

In a preferred embodiment of the use of the strain to produce biofuels, the strain produces lipids, including in particular triacylglycerol.

In a preferred embodiment of the use of the strain to produce biofuels, lipid production is stimulated by rapamycin.

The present invention refers to the strain of unicellular red algae *Cyanidioschyzon merolae* 10D, deposited on October 25, 2019 in accordance with requirements of Budapest treaty in Banco Español de Algas, Las Palmas, Spain, under the number BEA_IDA_0073B. This species in the natural environment is found in acidic, hot springs at pH below 2 and at a temperature of 42 - 65°C.

As indicated above, the novel strain of the invention is capable of effective growth, with a buffering agent, in the range of pH 5.5 - 6.7.

The buffering agent can be any buffering agent known in the art suitable for the given pH range, provided that it is not cytotoxic for the strain cells. The person skilled in the art is able to check the buffering agent for its cytotoxicity following standard methods known in the art, such as analysis of survivability and cell growth in the presence of the buffering agent, and to select an appropriate buffering agent without undue experimentation. An example of a preferred buffering agent may be MES buffer (based on 2-morpholinoethanesulfonic acid). Another exemplary buffering agent could be, e.g. Bis-Tris as a buffering agent effective in the pH range of 5.8-7.2.

An advantage of the strain according to the invention are the possibilities of using techniques for genome and transcriptome manipulation and analysis for the *C*. *merolae* organism, which until now have been significantly hampered due to too low pH. Under the utilized culture conditions of low pH (pH 2.5 for standard laboratory culture of *C*. *merolae*) DNA and mRNA are hydrolyzed, which compels the use of additional material preparation steps, reduces the efficiency of the process, extends its duration and increases the costs of the molecular methods used. When using a higher pH, difficulties associated with the research and manipulation of DNA and RNA are negligible. Importantly, when adapting the strain from standard pH range of 2.5 to higher pH of 6.7, said model organism may provide a great deal of valuable information on unique metabolic pathways, activated upon adaptation to environmental stressors.

The newly obtained strain of the invention is capable of biofilm formation on cotton fibers, in contrast to the control strain cultivated at pH 2.5. It is significant, because bioremediation using biofilms instead of cell suspensions is particularly effective in the treatment of groundwater and soil contaminated with heavy metals (Arindam Mitra, Suman Mukhopadhyay, Biofilm mediated decontamination of pollutants from the environment, AIMS Bioengineering, 2016, 3(1): 44-59). The presence of biofilm matrix facilitates precipitation of metals. In addition, a biofilm may constitute a barrier limiting the outflow of contaminants from a given place, limiting their spread.

An important aspect of the invention is the possibility of using the novel strain for removing heavy metals, phosphates and nitrates, i.e., in the broadly understood field of bioremediation. Currently, green algae have a wide range of applications in this field. It should be mentioned here that pH values and temperature are some of the most important parameters, determining the ability to capture heavy metal ions from the environment by the algae (Lee, Ming-Kuo & Saunders, James. (2003). Effects of pH on Metals Precipitation and Sorption: Field Bioremediation and Geochemical Modeling Approaches. Vadose Zone Journal - VADOSE ZONE J. 2. 177-185. 10.2113/2.2.177). Scientific publications indicate that when using higher pH values, as well as higher temperatures, the biosorption of metal ions by algae is more efficient, especially in the case of biofilms. Problematic in this case is the fact that using temperatures above 25°C for green algae causes their death, and consequently a decrease in sorption and bioremediation capabilities in general.

In the case of the obtained, stable *C*. *merolae* strain of the invention, viable both in a moderately high temperature (42°C) and a higher pH (pH 6.7), the capability of the novel strain to capture heavy metal ions increases. Possibility of cultivation at higher pH allows to optimize immobilization of the cells on solid surfaces, as well as handling aiming at increased biomass. The strain of the invention can also be employed in studies of metabolic pathways and molecular components, that underlie the increased adaptive potential of other algae strains, using molecular biology approaches (mutagenesis or RNA silencing). For example, synthesis of new proteins capable of binding and/or removing heavy metals can occur in *C*. *merolae* cells adapted to growth at a higher pH. The use of such proteins in higher plants for selective detoxification or accumulation of certain elements or compounds is an attractive prospect (Zeraatkar et al. Potential use of algae for heavy metal bioremediation, a critical review. J Environ Manage. 2016 Oct 1;181:817-831. doi: 10.1016/j.jenvman.2016.06.059).

The strain of the invention can be used in the so-called High Rate Algal Pond (HRAP), which enables more effective wastewater disinfection, low-energy treatment thereof, as well as biofuel production from the increased algae biomass obtained (R Craggs, J Park, S Heubeck & D Sutherland (2014) High rate algal pond systems for low-energy wastewater treatment, nutrient recovery and energy production, New Zealand Journal of Botany, 52:1, 60-73, DOI: 10.1080/0028825X.2013.861855).

The strain of the invention can be used for treatment of mine outflows of variable pH, as well as sulphur-containing wastewater from a heating and power plant characterized by an increased water temperature.

The strain of the invention can be used for removing biogenous contaminants (containing nitrogen and phosphorus compounds), contributing to eutrophication.

Additionally, studies are conducted on the use of *C*. *merolae* for efficient lipid production towards biotechnological applications, such as biofuel production. In general, under conditions of stress, algae produce more lipids. Until now, lipid production in *C*. *merolae* was examined only using standard conditions of laboratory cultures (Mori, N., Moriyama, T. and Sato, N. (2019), Uncommon properties of lipid biosynthesis of isolated plastids in the unicellular red alga Cyanidioschyzon merolae. FEBS Open Bio, 9: 114-128. doi:10.1002/2211-5463.12551), and the use of metabolic manipulation (use of rapamycin to inhibit the TOR kinase with the aim to increase cytosol lipid levels, including triacylglycerol, in *C*. *merolae* cells) in standard culture conditions was possible only after utilizing time-consuming and expensive genetic engineering manipulations (Imamura, S., Kawase, Y., Kobayashi, I. et al. Plant Mol Biol (2015) 89: 309. https://doi.org/10.1007/s11103-015-0370-6).

The studies carried out by the present inventors by means of quantitative measurement of lipids with a colorimetric method indicate about 16% lipids present in dry mass of a sample of *C*. *merole* strain according to the invention adapted to pH 6.5. As compared to *C*. *merolae* culture carried out at pH 2.5, the lipid content in the strain of the invention is similar. However, due to adapting the *C*. *merolae* culture to higher pH, utilization of the TOR kinase inhibition by rapamycin is facilitated, which is crucial for an increase of triacylglycerol production, which forms cytoplasmic lipid droplets in microalgae cells (Imamura et al. 2015, Plant Mol Biol (2015) 89:309-318 DOI 10.1007/s11103-015-0370-6). Rapamycin undergoes degradation at low pH, hence at increased pH it will be possible to use the above described simple metabolic manipulation (inhibition of the TOR kinase with rapamycin) for the first time also in a genetically non-modified *C*. *merolae* strain, without the need for time-consuming and expensive genetic manipulations described in Imamura et al. (2015).

Also disclosed but not part of the invention is a method for adapting a strain to the conditions of higher pH (up to 6.7) in a specific temperature range (25 - 45°C). The methods for adapting include the following steps: selecting suitable, healthy cells for passage, using appropriate medium, adapting the cells during the consecutive days to the target pH.

The method above involves using a starter culture, and applying small adaptive steps. The essence of the method above is a slow adaptation of the cells to a changing pH stimulus by diluting the dense culture with a small amount of culture medium of gradually increasing pH (starting from the first step of increasing the culture pH to 3.5, and then in each consecutive step of diluting the dense culture with a fresh medium, maintaining the increase of the culture pH of ±1 pH unit. It is very important to maintain the culture subjected to adaptation to the new pH for about 7 days to enable efficient metabolic adaptation of the cells (in that time an increase in cell number and a small decrease in the culture pH of 0.5 pH unit on average is observed). The exact time may vary depending on the culture conditions, including temperature, light intensity and CO₂ concentration in the air. For example, the culture growth rate may be increased by using a 5% CO₂ mixture in the air or by increasing a light intensity to 90 µE/m²/s; and in turn it may be decreased when the temperature of the culture is lowered. The person skilled in the art will be able to adjust conditions of the culture and passage duration to the specific capabilities and needs, without undue experimentation. Subsequent dilutions with further increases in pH may be done, for example, when the culture reaches OD₇₅₀~ 1.0 or higher. A systematically gradual adaptation method, is the key to maintaining high viability of the cells.

### Brief description of the figures

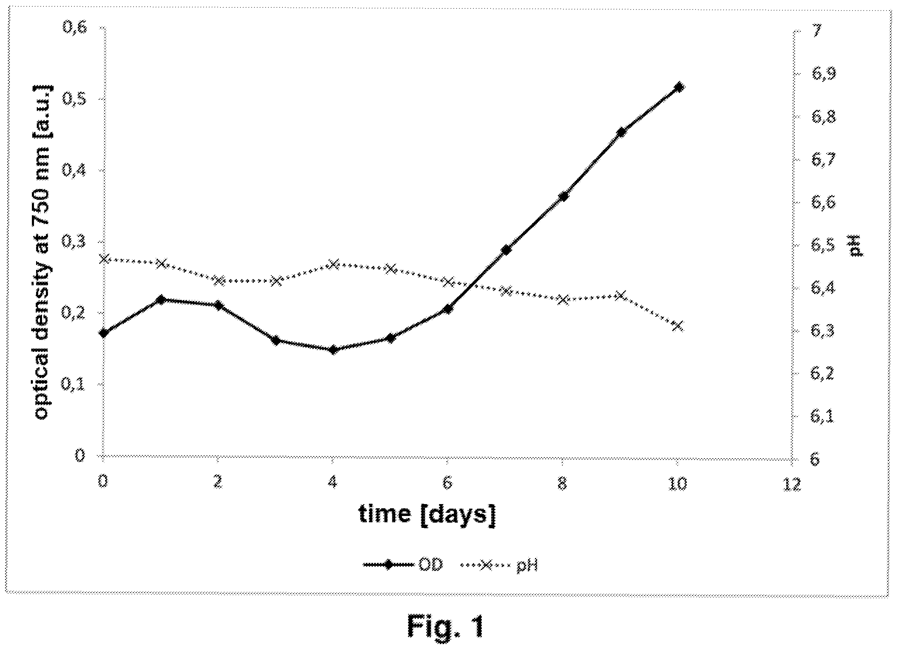

**Fig. 1****.** Growth curve of *C*. *merolae* cell suspension after adaptation to pH > 6.0. Additionally, pH changes are shown during 10-day cell culturing.

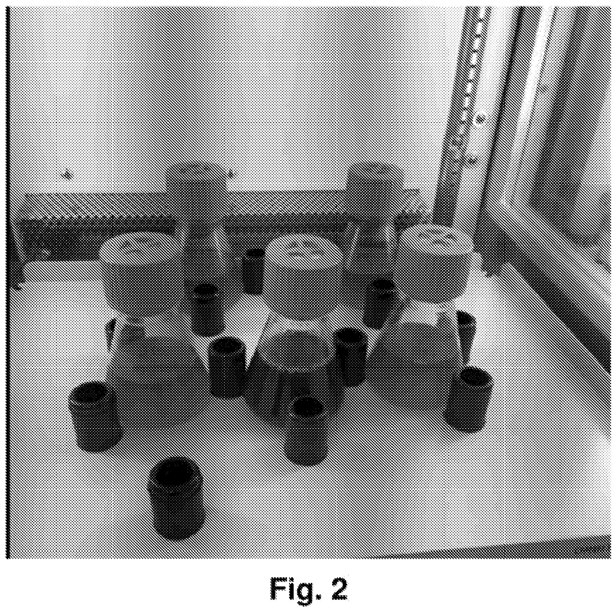

**Fig. 2****.** Representative cell suspension cultures of the stable *C*. *merolae* strain adapted to growth at pH 6.7.

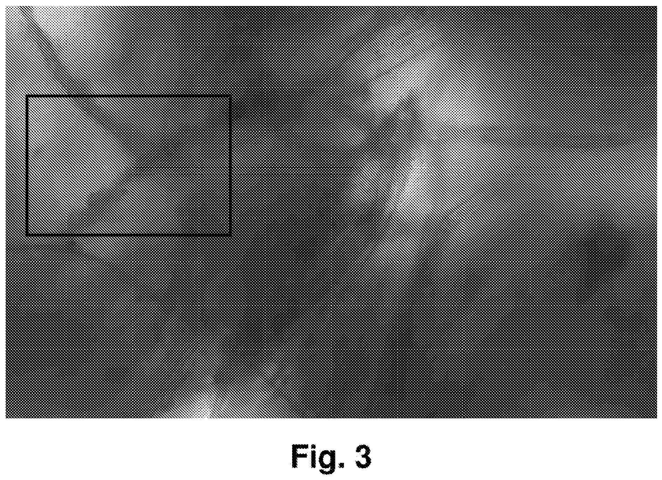

**Fig. 3****.** Micrograph of the stable *C*. *merolae* strain obtained according to the invention in the presence of cotton fibres. The biofilm forming on fibres is indicated, formed of *C*. *merolae* cells cultured at pH > 6. Micrographs were obtained at a 40x magnification.

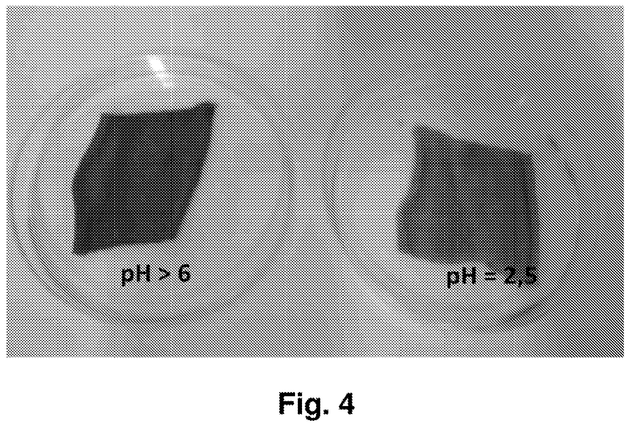

**Fig. 4****.** Biofilm formed by the cells of the novel *C*. *merolae* strain grown on cotton fabric substrate at pH > 6 (left) and biofilm of the control *C*. *merolae* strain grown in the standard pH of 2.5 (right). Density of the cells in the biofilm at pH > 6 is ~ 5-fold higher compared to *C*. *merolae* biofilm at pH 2.5 at the same analysed timepoint of the biofilm formation (7 days).

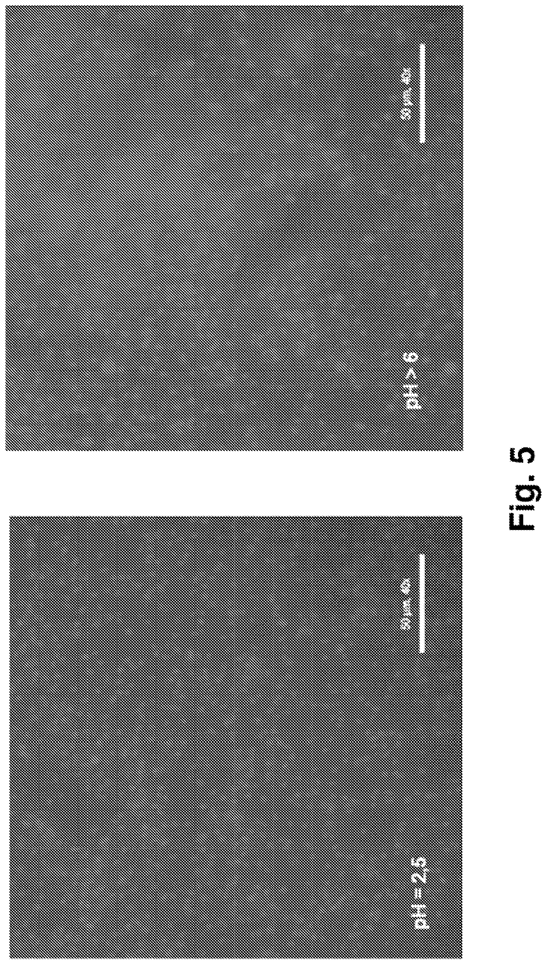

**Fig. 5****.** Fluorescent microscopy imaging of chlorophyll emission from the *C*. *merolae* cells grown at pH 2.5 (control, left) and pH > 6 (right). Micrographs were obtained following chlorophyll excitation at 580-598 nm using an LS720 fluorescence microscope (Etaluma) at 40x magnification. Scalebar: 50 µM. Both microphotographs show fluorescence of individual chloroplasts in *C*. *merolae* cells, demonstrating cell viability in both cultures.

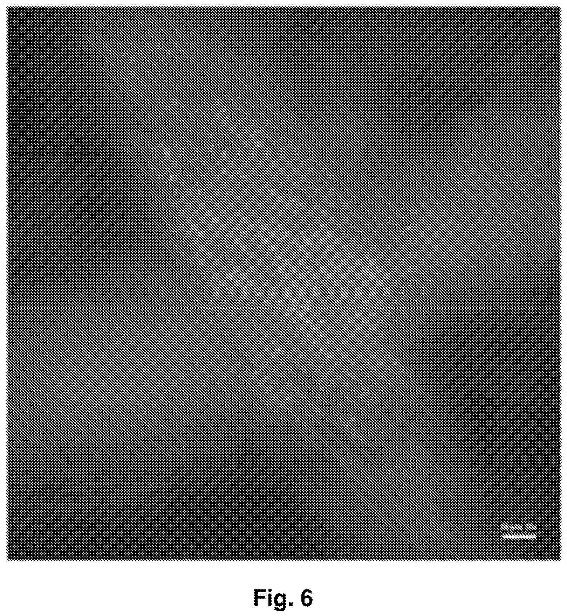

**Fig. 6****.** Fluorescence imaging of chlorophyll emission of single chloroplasts present inside *C*. *merolae* cells grown at pH > 6, forming a biofilm on cotton fibre substrate. Scalebar: 50 µm. Micrographs were obtained following chlorophyll excitation at 580-598 nm using an LS720 fluorescence microscope (Etaluma) at 40x magnification.

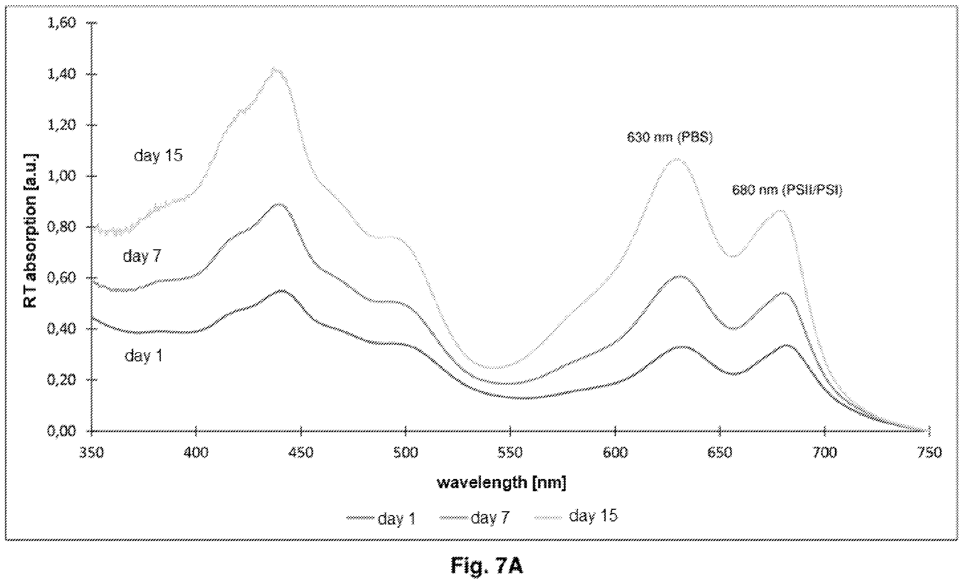

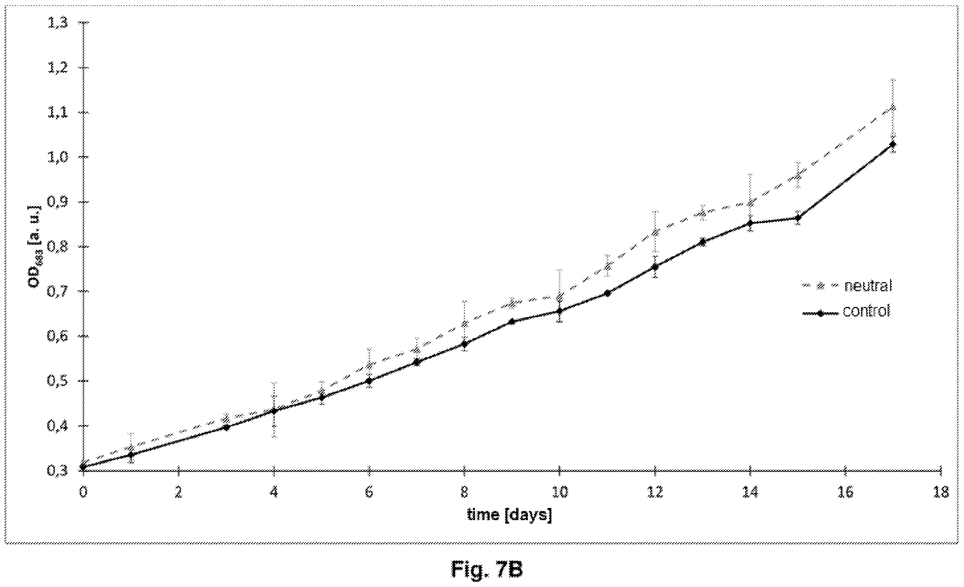

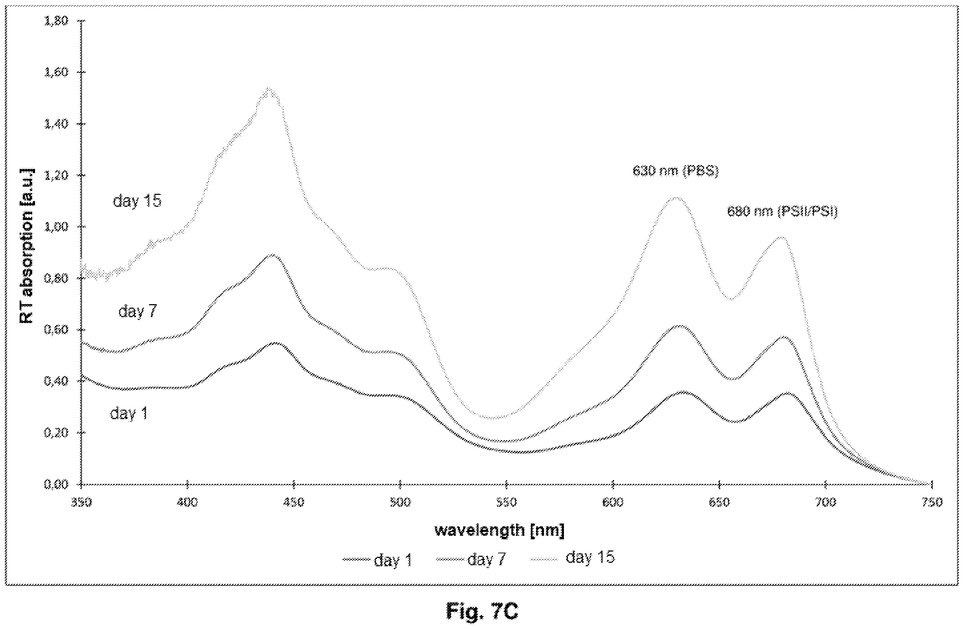

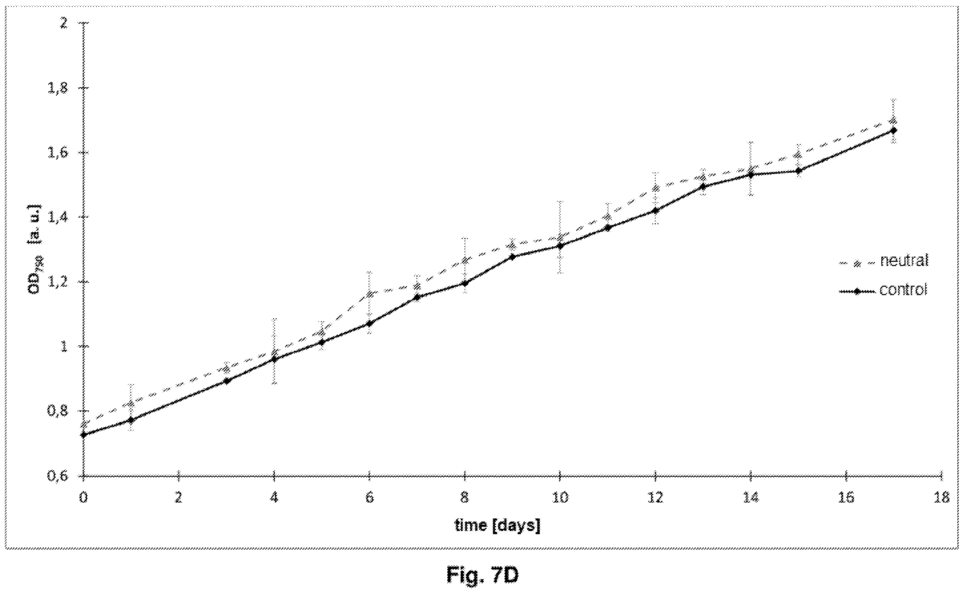

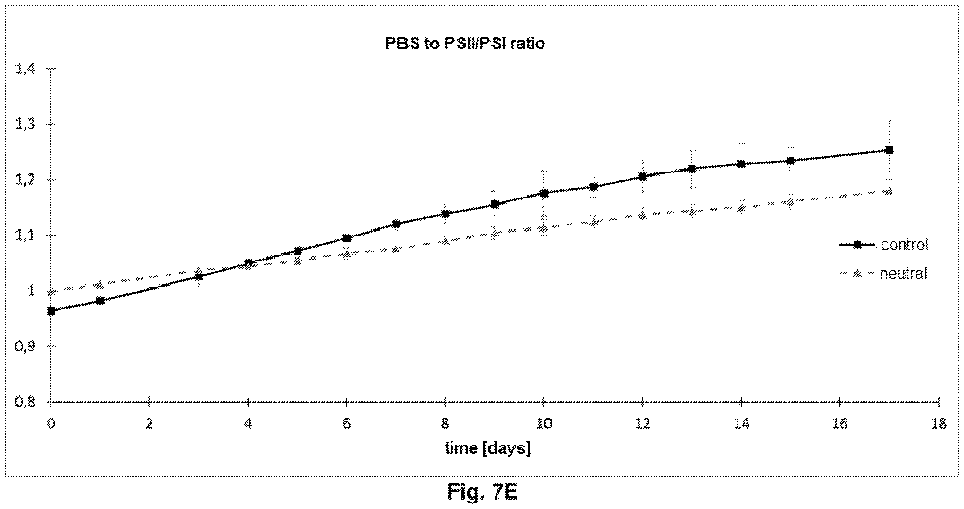

**Fig. 7** shows results obtained with RT absorption spectroscopy of the *C*. *merolae* control strain **(****Fig. 7A****)** and the novel *C*. *merolae* strain adapted to growth at neutral pH **(****Fig. 7C****).** For determination of the growth curves, optical density (OD) for cell cultures of both strains was measured at 683 nm **(****Fig. 7B****)** and 750 nm **(****Fig. 7D****).** **Fig. 7E** shows the ratio of the absorbance peak value corresponding to phycobilisomes (630 nm, PBS) and photosystems I and II (680 nm, PS) for each time point of the growth curves. Shown are the mean and standard deviation (SD) values obtained from two replicas of 2 independent biological samples (n=4).

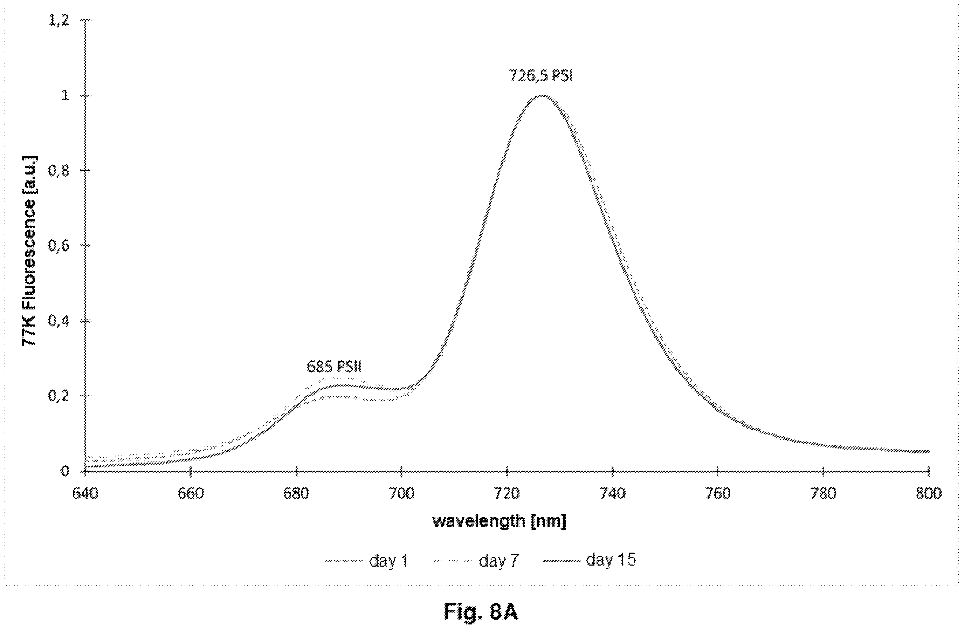

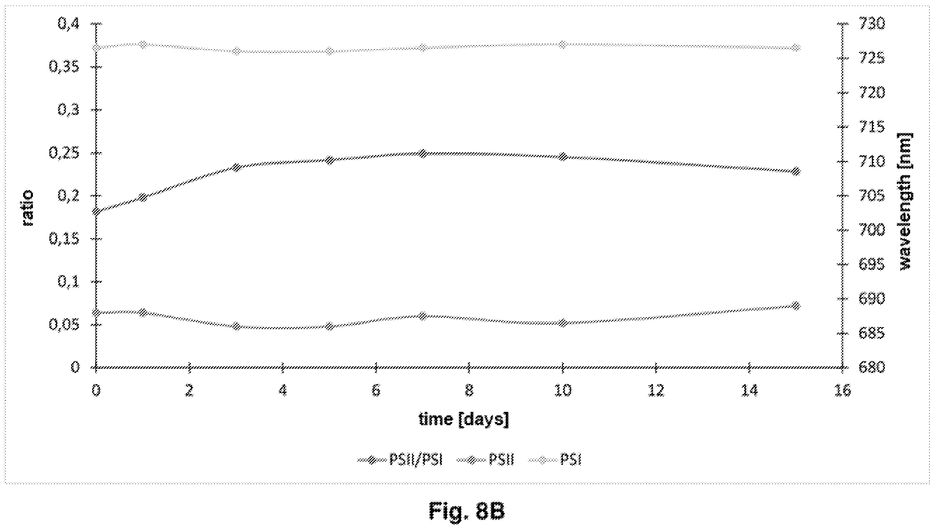

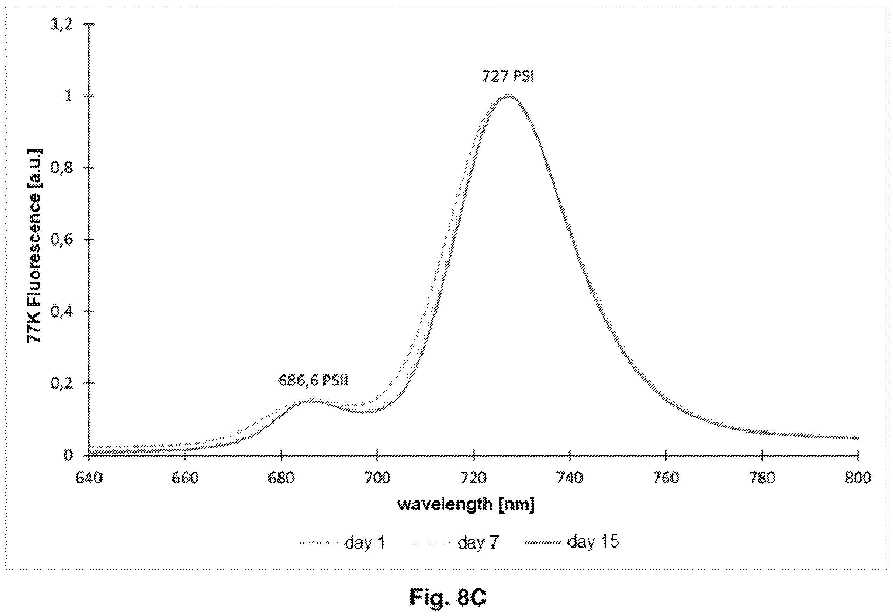

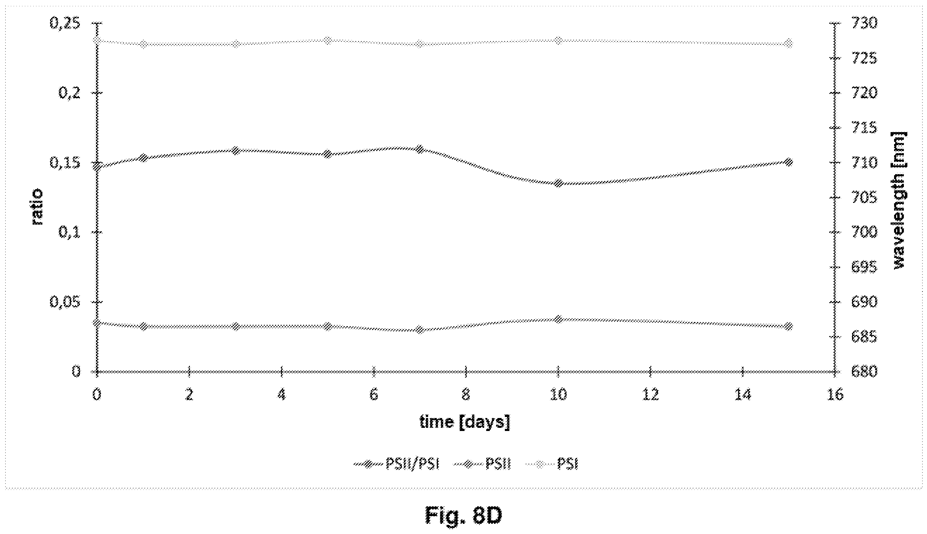

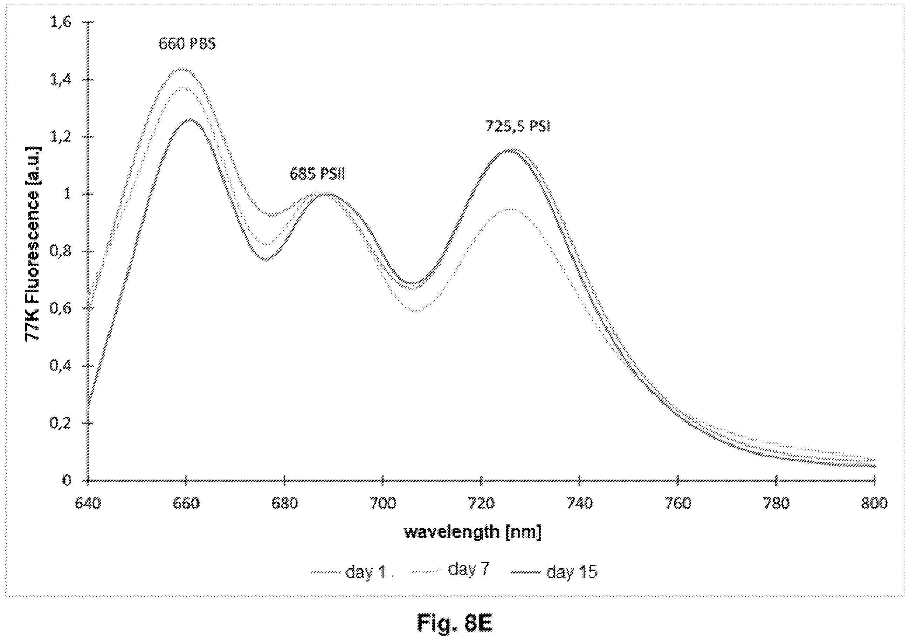

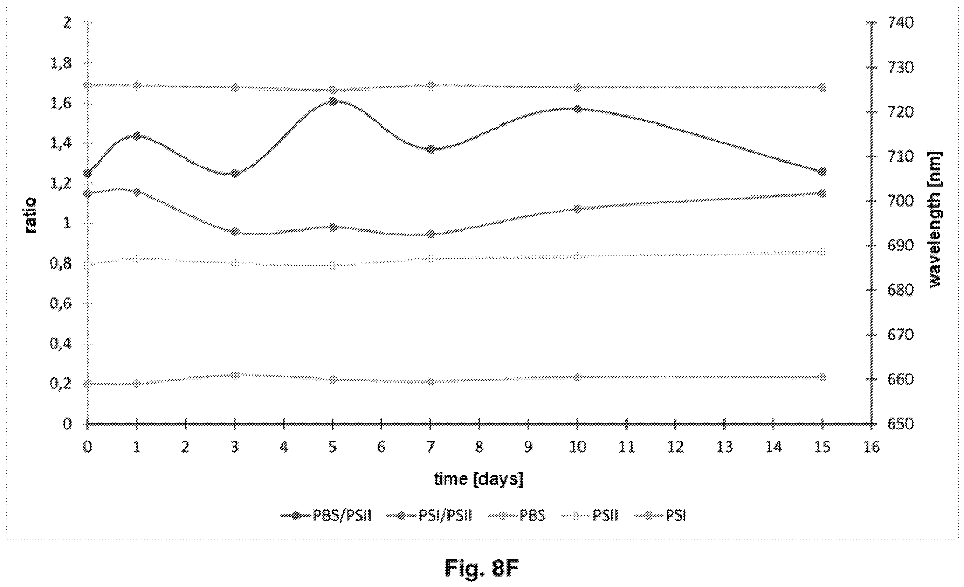

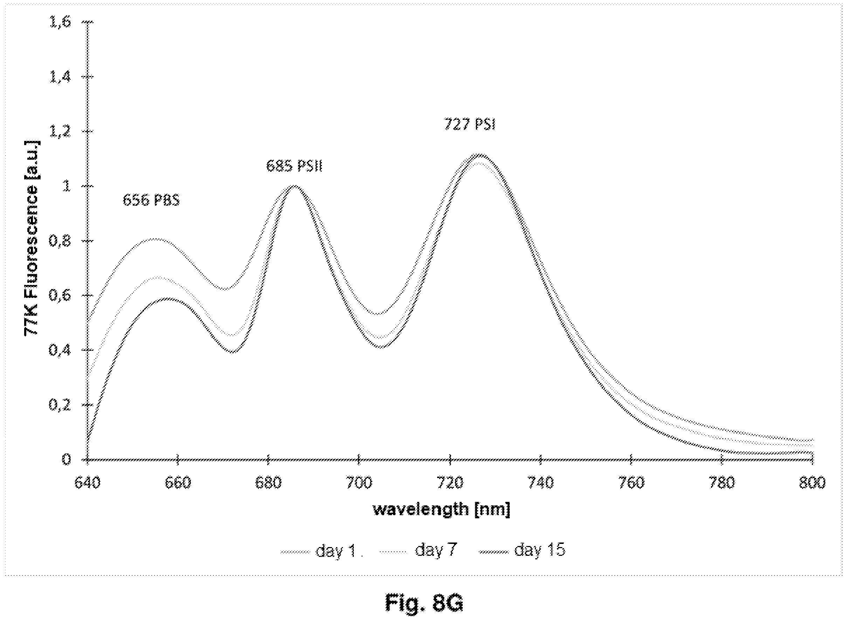

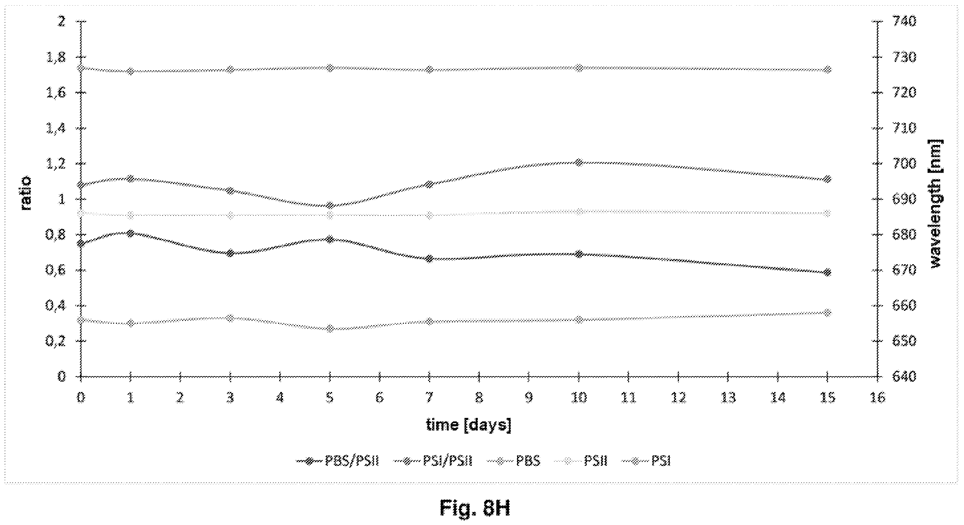

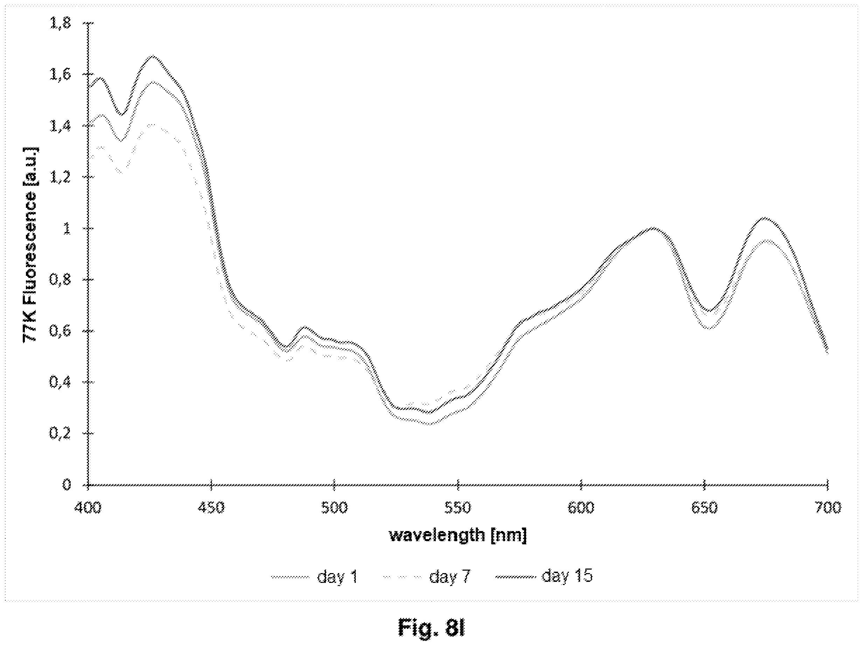

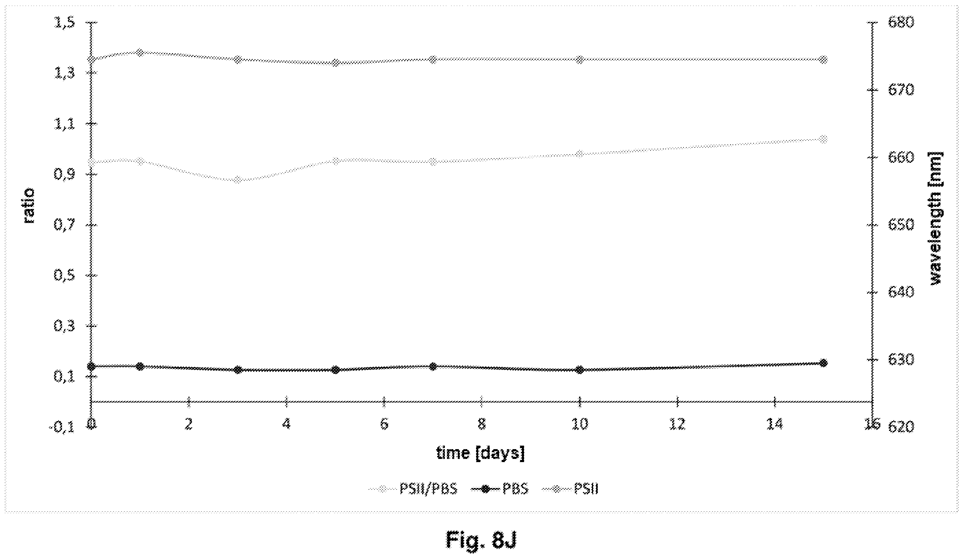

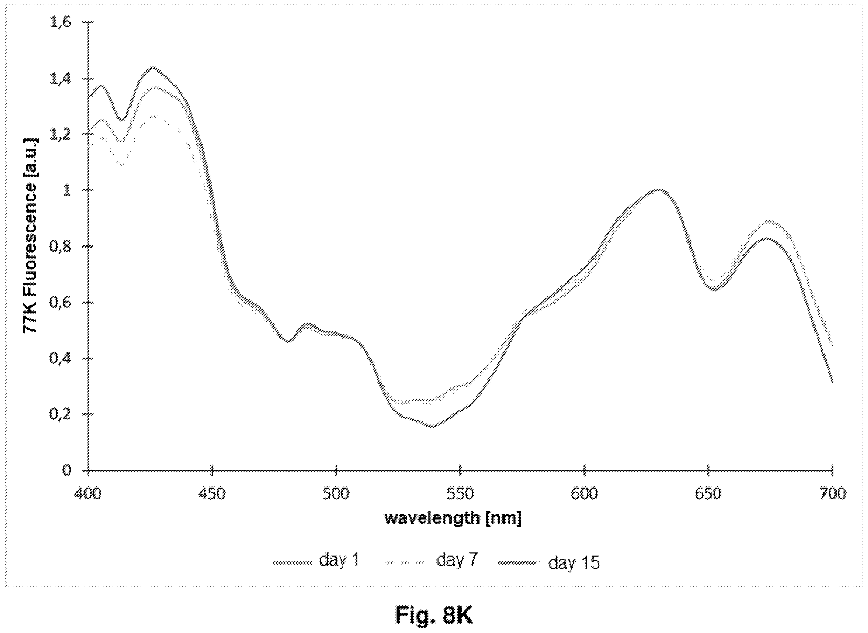

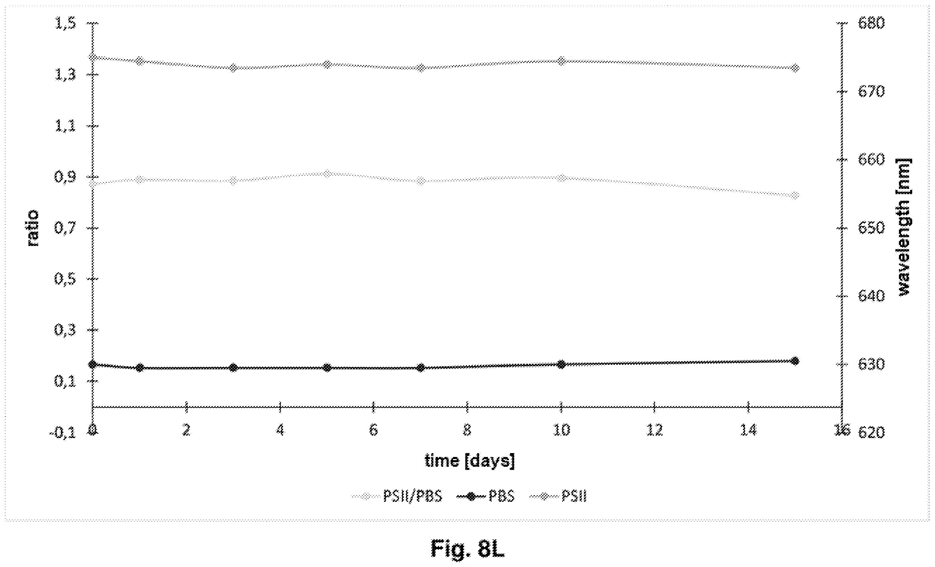

**Fig. 8** shows the results of 77K fluorescence spectroscopy of the *C*. *merolae* control strain (Fig. 8A, 8B, 8E, 8F, 8I, 8J) and the novel *C*. *merolae* strain adapted to neutral pH (Fig. 8C, 8D, 8G, 8H, 8K, 8L). Fig. 8A-8H display emission spectra following Chl excitation at 435 nm (Fig. 8A-8D) and PBS excitation at 600 nm (Fig. 8E-8H). Fig. 8I-8L show excitation spectra recorded at 728 nm. Spectra in Fig. 8A and 8C are normalised to the PSI emission peak, spectra in Fig. 8E and 8G are normalised to the PSII emission peak, while spectra in Fig. 8I and 8K are normalised to the PBS emission peak. Ratios of the emission peaks corresponding to phycobilisomes (PBS) and photosystems I (PSI) and II (PSII) are shown in Fig. 8B, 8D, 8F, 8H, 8J, 8L, respectively.

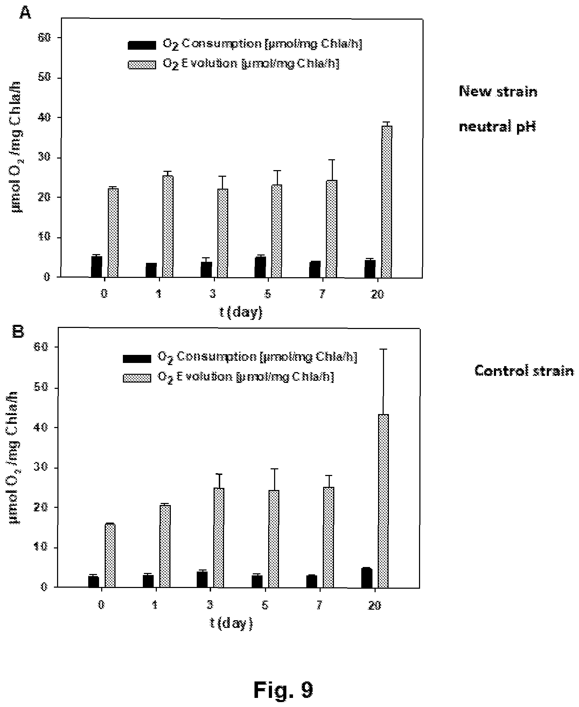

**Fig. 9** shows the activities for oxygen evolution (light) and consumption (dark) of the novel *C*. *merolae* strain in neutral pH. The cells were grown at pH 2.5 (control) or pH 6.8 (neutral pH). Cells in a logarithmic growth phase (OD₆₈₂ of 0.4) were incubated in the dark for 2 min. before each measurement with a Clark-type electrode. The data is mean values ± SD for 2 replicas of 2 independent biological samples (n=4). Oxygen evolution was measured under white light illumination of 2,000 µE/m²/s.

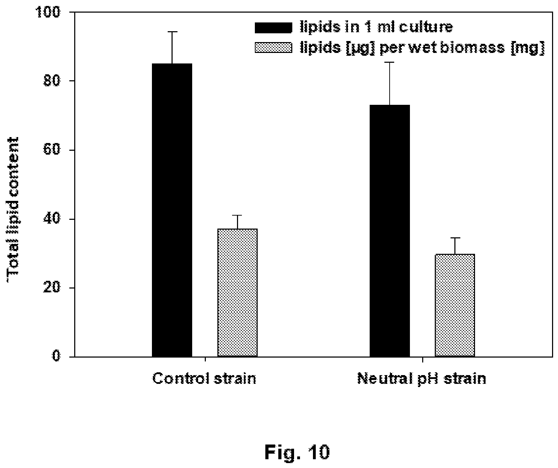

**Fig. 10** shows lipid content in the cells of the novel *C*. *merolae* strain at neutral pH. The data for total lipid content per 1 ml of cell suspension (OD₆₈₂ ~1.0) or per mg of fresh mass for the control and the strain in neutral pH are mean values ± SD for 2 replicas of 2 independent biological samples (n=4).

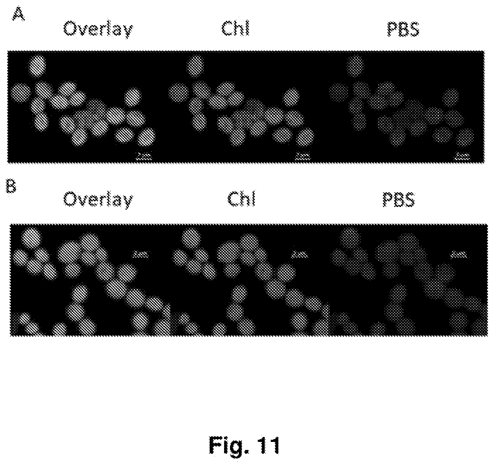

**Fig. 11** shows confocal microscopy imaging for the *C*. *merolae* control (A) and the novel *C*. *merolae* strain at neutral pH (B). Mean cell size for the control and the cells adapted to neutral pH strain was estimated as 3.88 and 6.02 µm, respectively. Excitation wavelengths were 488 nm (PSI and PSII Chl emission) and 555 nm (PBS emission), respectively. Scalebar corresponds to 2 µm.

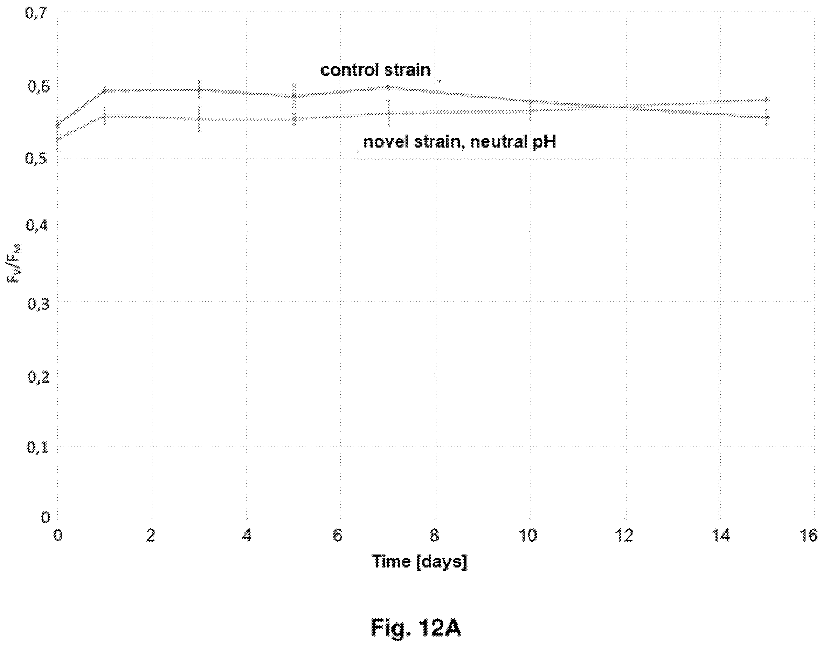

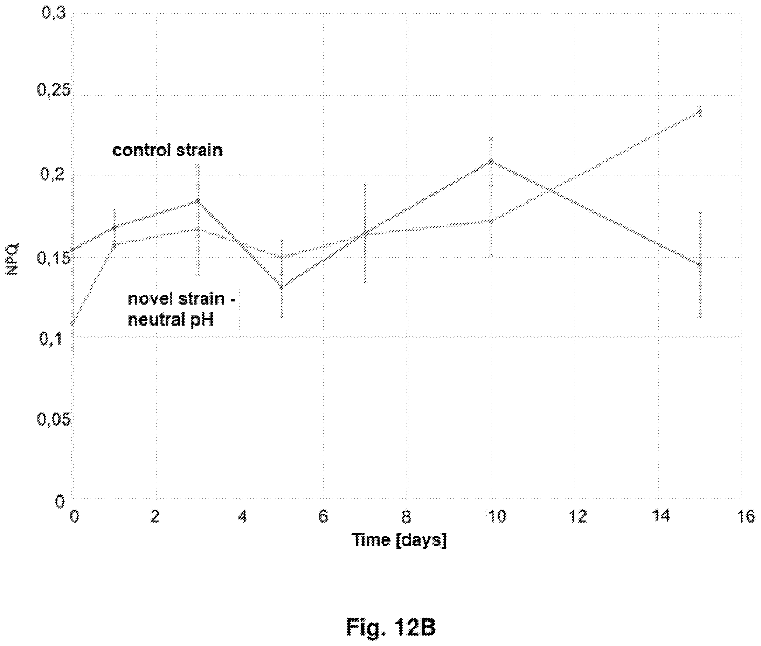

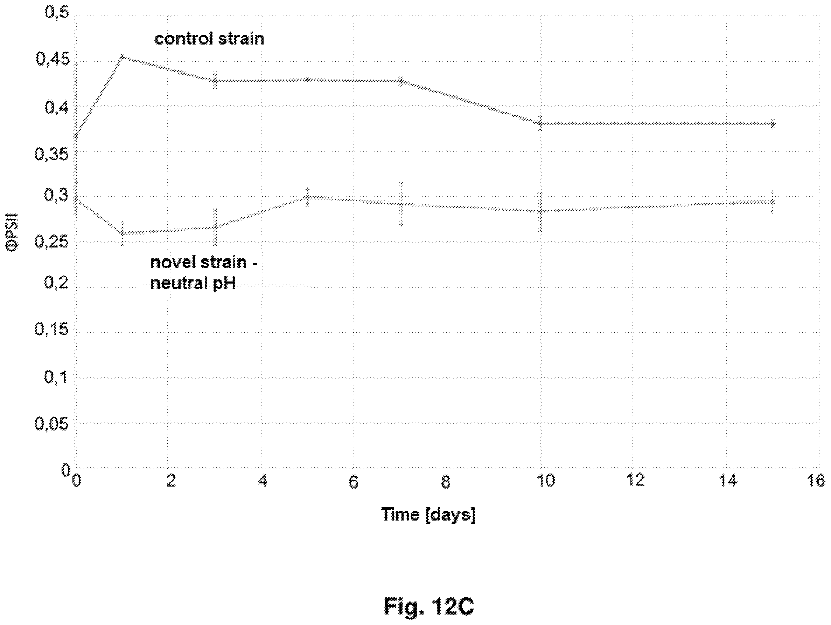

**Fig. 12** shows pulse amplitude-modulated (PAM) fluorimetric analysis for the *C*. *merolae* control strain cells and for the cells of the novel *C*. *merolae* strain adapted to neutral pH. Shown are: the maximum PSII efficiency (Fᵥ/Fₘ ratio, Fig. 12A), nonphotochemical quenching (NPQ, Fig 12B) and photochemical efficiency for PSII (ϕPSII, Fig. 12C). Data represent mean values ± SD of 2 independent biological samples (n=2).

### EXAMPLES

### Example 1. Preparation of the novel Cyanidioschyzon merolae strain adapted to growth in variable pH conditions.

Unicellular red algae (red microalgae) - *Cyanidioschyzon merolae,* the strain from the NIES-1332 collection, for which standard control culture conditions are: M-Allen medium × 2 (pH 2.5), 42°C, 5% CO₂, continuous white light, 90 µE (µmol_{phot.}*m⁻²*s⁻¹), was adapted to variable pH conditions, where the highest recorded value was 6.7. During adaptation high flexibility of the novel strain was demonstrated, in relation to the applied diverse pH values.

For cultivation of the novel strain the following procedures were applied:
1. Culture medium as above with modifications ensuring higher pH.
Sulfuric acid (VI) H₂SO₄ was used to adjust the pH for pH values < 3.5 and sodium hydroxide NaOH for pH values > 3.5, because pH of the medium before addition of H₂SO₄ is ~3.5. In the method, successive portions of the dilution medium with increasingly higher pH were used.
2. Culture medium with increased pH additionally containing MES buffer (2-morpholinoethanesulfonic acid) (CAS: 4432-31-9) 10 or 20 mM.

| M-Allen x2 (1L) | |
|---|---|
| MES | 10 mM or 20 mM |
| (NH₄)₂SO₄ | 20 mM |
| KH₂PO₄ | 4 mM |
| MgSO₄ · 7H₂O | 2 mM |
| CaCl₂ · 2H₂O | 1 mM |
| A2 trace stock | 2 mL |
| After autoclaving | |
| A2 Fe stock solution | 4 mL |
| | |

| A2 trace element stock solution (100 mL) | |
|---|---|
| MnCl₂ · 4H₂O | 910 mM |
| H₃BO₃ | 461 mM |
| Na₂MoO₄ · 2H₂O | 161 mM |
| ZnCl₂ | 77 mM |
| CuCl₂ · 2H₂O | 25 mM |
| CoCl₂ · 6H₂O | 17 mM |
| | |

| A2 Fe stock solution (100 ml) | |
|---|---|
| (filtered, non-autoclaved) | |
| EDTA · 2Na · 2H₂O | 208 mM |
| FeCl₃ | 148 mM |

Next, the medium was sterilized by autoclaving.

To the M-Allen medium × 2, after autoclaving, an iron source, so-called "Fe stock solution", was added, which was previously prepared in sterile conditions and filtered.

Successive steps of the experiment leading to the novel strain:
1. Starter culture: *C*. *merolae* strain NIES-1332 (OD₇₅₀ ~ 1, pH 2.6)
2. Preliminary series of passages to medium without MES, with increased pH. Dense culture was diluted with a small amount of the culture medium, starting from pH of the culture of about 3.5. After the growth period to OD₇₅₀ of about 1.0 (approx. 7-12 days, time depends on the culture conditions and culture density) another passage was carried out to a small amount of another, fresh portion of the culture medium of higher pH. The passages were performed so as to keep the increase in the culture's pH of about 1 pH unit per passage. The passages at this stage were carried out until culture pH was 5.5. Passage was made when the donor culture density was sufficient to provide a suitably large number of cells after passage (dense culture was found to better withstand the stress of pH change).
3. pH stabilizing passages with an addition of a buffering agent, MES 10-20 mM, e.g., 10 mM, so as to maintain an increase in the culture's pH of about 0.5 pH unit per passage.
4. pH stabilizing passages with an addition of a buffering agent, MES 10-20 mM, e.g. 20 mM, so as to obtain the final pH of the culture of ± 6.7.
5. Final culture with stable pH (6.5 - 6.8).

Successive steps of the experiment which yield the novel strain (detailed description of the experiment):
For preliminary passage to higher pH the following procedures were used:
- starter culture with stable growth, at OD₇₅₀ ~ 1, pH 2.6 (pH adjusted with sulfuric(VI) acid.
- modified M-Allen medium × 2, without MES with pH ~ 6.5 (pH adjusted with sodium hydroxide, NaOH)

The starter culture was inoculated into the M-Allen medium × 2 with an increased pH. Since no MES buffering agent is used in the first passages, the appropriate pH must be determined by measuring with a pH-meter by mixing the starter culture and M-Allen medium × 2 (higher pH) under sterile conditions at a burner. However, in this case, the pH of the M-Allen medium × 2 should be as high as about 7.7. Below an example of adjusting the pH of the culture medium without the use of a buffering agent is provided. A critical part of the procedure is to obtain appropriate pH after passage:
M-Allen medium × 2: pH adjustment before autoclaving and exemplary pH values:

| Test no. | Before addition of Fe stock solution | After addition of Fe stock solution |
|---|---|---|
| 1 | 7.03 | 6.94 |
| 2 | 7.64 | 7.52 |

The following were used for the experiment: sterile 750 mL/175 cm² bottles for the suspension culture, with a Plug-Seal screw cap, Nest Scientific Biotechnology. The bottles with the culture were shaded with aluminum foil for the first 3-4 days of cultivation in a chamber or light of low intensity 30 µE (µmol_{phot.}*m⁻²*s⁻¹) was applied. Cell cultivation was performed in thermostatic Panasonic incubator (model MLR-352) at a constant temperature of 42°C and white light intensity gradually increased from 30 µE to 90 µE (µmol_{phot.}*m⁻²*s⁻¹), depending on the optical density of the culture monitored at 750 nm (optical density at 750 nm, OD₇₅₀) throughout the duration of the culture. The source of white light were lamps of GrowLight type (PANASONIC FL40SS ENW/37 type).

### Passage (1)

| No. | Day of culture | pH value before starter culture addition | Amount of M-Allen medium x 2 of higher pH) | Amount of starter culture added (from acidic pH 2.61) | New pH | New OD₇₅₀* |
|---|---|---|---|---|---|---|
| 1 | start | 6.94 | 75 mL | 25 mL | 3.57 | 0.3291 |
| 2 | start | 7.52 | 75 mL | 27.5 mL | 5.45 | 0.3677 |

| | | | | | | |
|---|---|---|---|---|---|---|
| *The new culture should not display OD₇₅₀ lower than 0.3. | | | | | | |

For the donor culture of OD₇₅₀ > 1.4, the new culture should display OD₇₅₀ > 0.01*E (where E is PAR intensity in µmol_{phot.}*m⁻²*s⁻¹) - correlation between initial OD₇₅₀ and illumination intensity.

| No. | 1 | 2 |
|---|---|---|
| Day of culture | 3 (72h) | 3 (72h) |
| OD₇₅₀ | 0.4507 | 0.4781 |
| pH | 3.43 | 4.11 |
| Day of culture | 7 | 7 |
| OD₇₅₀ | 0.7361 | 0.7487 |
| pH | 3.21 | 3.56 |
| Day of culture | 12 | 12 |
| OD₇₅₀ | 1.0229 | 1.0521 |
| pH | 3.01 | 3.24 |

At OD₇₅₀ ~ 1.0 another passage to fresh medium without MES buffering agent should be done.

### Passage (2)

| No. | Day of culture | pH value for cultivation after day 12 (from passage 1) Donor culture | Amount of M-Allen medium x 2, pH 7.0) | Amount of culture added from previous passage | New pH | New OD₇₅₀* |
|---|---|---|---|---|---|---|
| 1 | start | 3.01 | 75 mL | 75 mL | 4.02 | 0.5655 |
| 2 | start | 3.24 | 75 mL | 75 mL | 5.45 | 0.5953 |

| No. | 1 | 2 | |
|---|---|---|---|
| Day of culture | 3 (72h) | 3 (72h) | |
| OD₇₅₀ | 0.7681 | 0.7762 | |
| pH | 3.60 | 3.98 | |
| Day of culture | 8 | 8 | →donor |
| OD₇₅₀ | 1.1160 | 1.1648 | |
| pH | 3.31 | 3.35 | |

### Passage (3)

In this passage, buffering agent MES 20 mM was used for the first time, but it was used only in the culture No. 2 for comparison:
Culture No. 1: passage in the 1:1 ratio; dilution factor of the donor culture with a fresh culture medium (without MES) is 2x; the new pH of the cell culture is 5.19.
Culture No. 2: passage to M-Allen medium × 2, pH 5.7 (with MES 20 mM); dilution factor of the donor culture with a fresh culture medium is 2.8x; the new pH of the cell culture is 5.6.

| No. | 1 | 2 |
|---|---|---|
| Day of culture | 3 (72h) | 3 (72h) |
| OD₇₅₀ | 0.7864 | 0.6592 |
| pH | 3.98 | 5.26 |
| Day of culture | 9 | 9 |
| OD₇₅₀ | 1.1777 | 1.2416 |

Subsequent passages at OD₇₅₀ ~ 1.0 were already carried out using M-Allen medium × 2 with 20 mM MES.

At the passage 5 - 6, a stable culture with pH about 6.0 is obtained, then from that point on, passaging to M-Allen medium × 2 (with 20 mM MES) with pH > 6.0 can commence.

Adaptive days are between 2 and 5 days. Fig. 1 shows the growth of the steadily growing culture at pH > 6.0. Fig. 2 shows a stable strain under laboratory conditions in a growth chamber. A photograph of the culture under the light microscope is shown in Fig. 3. Photographs of biofilms produced by the cells of the novel strain at pH > 6.0, compared to the biofilm produced by the control starter culture at pH 2.5, are shown in Fig. 4.

Viability of the culture of the novel strain was also assessed by analyzing individual C. *merolae* cells with the fluorescence microscope LS720 (Etaluma) at a magnification of 40 and chlorophyll excitation at 580-598 nm. Fig. 5 shows a comparison of the novel strain obtained and cultured at elevated pH (pH > 6), with a control strain (starter culture cultivated at pH 2.5), showing a comparable level of chloroplast fluorescence, and thus the viability of the culture. Fig. 6 shows fluorescence microscopy imaging of chlorophyll emission from individual chloroplasts present in the cells of the C. *merolae* strain obtained and cultivated at pH > 6, forming a biofilm on the cotton fiber substrate.

Lipid content for the strain obtained was also analyzed. Lipids were quantified with the colorimetric sulfo-phospho-vanillin (SPV) method at 526 nm. For cultures at pH 2.5 and 6.7, the amount of lipids in a fresh biomass was 16.03% and 16.46%, respectively.

### Additional information:

1. Note: when OD₇₅₀ starts to drop noticeably, and the cells begin to die, the culture should not be terminated, but better shading from light should be provided for the culture. After a few days (most often 3-4), when the main adaptation occurs, the cells should start to grow again. The reason for dying is usually due to too excessive dilution of the culture, with the pH increase being too high, in relation to the culture density.
2. It was observed that a longer adaptation may be done, by adding M-Allen medium × 2 (~ pH 6.5) with MES 10 mM buffering agent to the culture with increased pH, after the few first passages without MES.
3. The optimum temperature for cultivation is 42°C, but cultivation is possible at a different, for example, lower temperature (e.g., 25°C).
4. Growth cycle of the strain is variable, thus depending on the adaptation of the starter culture from pH 2.5 to the conditions of higher pH, the new culture may display a different growth rate in later passages.
5. Growing algae requires sterile conditions, especially for the steps of preparing the growth medium with higher pH.
6. The strain is autotrophic and requires automatic mixing in the chamber so that the cells do not settle to the bottom of the culture flasks.
7. The new strain has biofilm forming properties.
8. Adapting the strain to a new pH occurs fairly quickly, when appropriate key adaptation steps, mentioned above, are used.

### Example 2. Biochemical and spectroscopic characteristics of the novel C. merole strain adapted to growth at neutral pH

Fig. 7 shows the results obtained using RT absorption spectroscopy for the strains of *C*. *merolae:* control (Fig. 7A) and the novel strain adapted to neutral pH (Fig. 7C). To determine growth curves, optical density (OD) for both strains was determined at 683 nm (Fig. 7B) and 750 nm (Fig. 7D). Fig. 7E shows the ratio of the absorbance peaks corresponding to phycobilisomes (630 nm, PBS) and the photosystems I and II (680 nm, PS) for each time point of the growth curves. Data for growth curves are means ± SD for two replicas from 2 independent biological samples (n=4).

The growth curves for *C*. *merolae* strain obtained and described in Example 1, at neutral pH, monitored at two wavelengths (683 nm and 750 nm) (Fig. 7B i 7D) show that the novel *C*. *merolae* strain has similar cell growth dynamics as the control strain (Fig. 7B, 7D), and on day 18 of cultivation under the illumination of 90 µE/m²/s is in the so-called 'mid-log' phase of growth.

The content of the light harvesting antenna proteins functionally linked to the photosystem II (PSII, water photooxidation enzyme) called phycobilisomes (PBS) is generally lower (from day 5 of cultivation, Fig. 7E) for the novel strain adapted to living at neutral pH as compared to the control strain (culture at pH 2.5), which is most likely related to the forming of photoprotective adaptive mechanism in the cells of the novel strain. This mechanism (reducing the amount of PBS proteins functionally linked to PSII) allows the novel *C*. *merolae* strain to function better at higher illumination (above 1,000 µE/m²/s) through a change in PSII structure (reducing functional light-absorbing PSII antennas) ensuring minimization of the stress of high light intensity, and thus protection against photoinhibition of the photosynthetic apparatus under high light (see below for discussion of the results obtained by the PAM fluorescence measurements shown in Fig. 12).

New data on the fluorescence of PBS antenna proteins functionally linked to PSII as compared to the emission of photosynthetic complexes PSII and photosystem I (PSI) are shown in Fig. 7 and 8. Changes in the content of functional PBS antennas were monitored during the cultivation of *C*. *merolae* (a novel strain in neutral pH as compared to the control strain at pH 2.5) by spectroscopic measurements (absorbance spectra in Fig. 7 and low temperature fluorescence spectra in Fig. 8).

In addition, no functional binding of PBS to PSI was found in any of the two *C*. *merolae* strains examined, as shown by measurements of the excitation spectra recorded for wavelength of light absorbed by PSI (728 nm, Fig. 8K). These two independent research approaches confirmed the above-described trend to reduce the content of PBS antenna proteins functionally linked to PSII in the novel *C*. *merolae* strain adapted to living at neutral pH.

The novel *C*. *merolae* strain adapted to growth at neutral pH is characterized by an equally high activity of the water photo-oxidizing complex (PSII) as the control strain, as shown in Fig. 9. Likewise, the activity of cellular respiration is similar in the novel strain at neutral pH as compared to control (Fig. 9).

The total lipid content (as measured colorimetrically according to Mishra et al., 2014) is similar in the novel *C*. *merolae* strain adapted to growth at neutral pH compared to the control strain. It is on average 518 ±89 µg/mL of culture for the strain at neutral pH and 604 ±66 µg/ml of culture for the control strain (Fig. 10) at OD₆₈₂ = 1 for both cell type cultures.

Confocal microscopy showed that the cell size of the novel *C*. *merolae* strain adapted to growth at neutral pH is on average 1.6-fold larger as compared to that of control cells (Fig. 11). The pattern of photosynthetic membranes in individual chloroplasts of the cells of the novel strain is similar as in the control strain. Non-fluorescent intracellular structures in the cells of the novel strain were observed (Fig. 11B), which were not found in the cells of the control strain. It is assumed that these new intracellular structures represent lipid bodies.

The parameters of photochemical and non-photochemical efficiency of the cells of the novel strain were examined by comparing them with the control strain (Fig.12). The maximum quantum yield of the water photo-oxidizing enzyme, PSII (Fᵥ/Fₘ, Fig. 6A) is similar for the novel strain at neutral pH and control. The measured non-photochemical quenching parameter is similar for both strains (NPQ, Fig. 12B), while the photosynthetic efficiency (relative amount of light absorbed by the PSII complex, used for photochemical processes) is on average 20% lower in the cells of the novel strain at neutral pH as compared to control (ΦPSII, Fig. 12C). This observation along with a reduction in the number of PBS antenna complexes functionally linked to the PSII complex (Fig. 7 and 8) constitutes an adaptation mechanism induced in the cells of the novel *C*. *merolae* strain, which allows these cells to function better at high light intensities through minimizing over-excitation processes of the PSII complex. The above-mentioned reduction of the amount of PBS antenna proteins significantly facilitates cultivation of the novel strain in large volumes, by minimizing the negative effect of shading on the biomass increase of the large-scale cell culture, which is important for improving the application value of the novel strain.

### Literature

Mishra SK, Suh Wl, Farooq W, Moon M, Shrivastav A, Park MS, Yang J-W (2014) Rapid quantification of microalgal lipids in aqueous medium by a simple colorimetric method. Bioresource Technol, 155, 330-333.

## Claims

1. A strain of unicellular red algae *Cyanidioschyzon merolae,* deposited on 25^{th} October 2019 in accordance with the requirements of The Budapest treaty in Banco Español de Algas, Las Palmas, Spain, under the number BEA_IDA 0073B.

2. Use of the strain of claim 1 for bioremediation.

3. The use for bioremediation according to claim 2, **characterized in that** the bioremediation includes removal of heavy metals.

4. The use for bioremediation according to claim 2 or 3, **characterized in that** wastewater or groundwater are subjected to bioremediation.

5. Use of the strain of claim 1, for the production of biofuels.

6. The use for the production of biofuels according to claim 5, **characterized in that** the strain produces lipids, including in particular triacylglycerol.

7. The use for the production of biofuels according to claim 6, **characterized in that** the production of lipids is stimulated with rapamycin.

## Patentansprüche

1. Stamm der einzelligen Rotalge *Cyanidioschyzon merolae,* hinterlegt am 25. Oktober 2019 in Übereinstimmung mit den Anforderungen des Budapester Abkommens bei der Banco Español de Algas, Las Palmas, Spanien, unter der Nummer BEA_IDA_0073B.

2. Verwendung des Stammes nach Anspruch 1 zur Bioremediation.

3. Verwendung zur Bioremediation nach Anspruch 2, **dadurch gekennzeichnet, dass** die Bioremediation die Entfernung von Schwermetallen einschließt.

4. Verwendung zur Bioremediation nach Anspruch 2 oder 3, **dadurch gekennzeichnet, dass** Abwässer oder Grundwasser einer Bioremediation unterzogen werden.

5. Verwendung des Stammes nach Anspruch 1, für die Herstellung von Biokraftstoffen.

6. Verwendung zur Herstellung von Biokraftstoffen nach Anspruch 5, **dadurch gekennzeichnet, dass** der Stamm Lipide produziert, einschließlich insbesondere Triacylglycerin.

7. Verwendung zur Herstellung von Biokraftstoffen nach Anspruch 6, **dadurch gekennzeichnet, dass** die Produktion von Lipiden durch Rapamycin stimuliert wird.

## Revendications

1. Souche d'algues rouges unicellulaires *Cyanidioschyzon merolae,* déposée le 25 octobre 2019 conformément aux exigences du traité de Budapest auprès de la Banque espagnole d'algues, Las Palmas, Espagne, sous le numéro BEA_IDA_0073B.

2. Utilisation de la souche selon la revendication 1 pour une bioremédiation.

3. Utilisation pour la bioremédiation selon la revendication 2, **caractérisée en ce que** la bioremédiation inclut l'élimination de métaux lourds.

4. Utilisation pour la bioremédiation selon la revendication 2 ou la revendication 3, **caractérisée en ce que** des eaux usées ou des eaux souterraines sont soumises à une bioremédiation.

5. Utilisation de la souche selon la revendication 1, pour la production de biocarburants.

6. Utilisation pour la production de biocarburants selon la revendication 5, **caractérisée en ce que** la souche produit des lipides, incluant notamment du triacylglycérol.

7. Utilisation pour la production de biocarburants selon la revendication 6 **caractérisée en ce que** la production de lipides est stimulée avec de la rapamycine.
